# EUROPEAN PATENT SPECIFICATION

(11) **EP 3 757 776 B1**
(45) Date of publication and mention of the grant of the patent: **29.11.2023**
(21) Application number: 19305854.2
(22) Date of filing: 26.06.2019
(51) Int. Cl.: G06F 9/4401, G06F 8/656, A61M 5/142

(54) **METHOD FOR BOOTING A MEDICAL DEVICE**
VERFAHREN ZUM HOCHFAHREN EINER MEDIZINISCHEN VORRICHTUNG
PROCÉDÉ DE DÉMARRAGE DE DISPOSITIF MÉDICAL

(43) Date of publication of application: 30.12.2020
(73) Proprietor: Fresenius Vial SAS, 38590 Brézins (FR)
(72) Inventor: TIDLIOUINE, Rachid, 38000 Grenoble (FR)
(74) Representative: Maikowski & Ninnemann Patentanwälte Partnerschaft mbB

(56) References cited:
- WO-A1-01/52935
- US-A1- 2019 187 970

## Description

The invention relates to a method for booting a medical device according to the preamble of claim 1 and to a medical device.

Within a method of this kind, a bootloader software is executed for booting the medical device, the bootloader software being stored in a first memory of a memory device of the medical device.

The booting of a medical device generally is understood - as for a generic computing device - as the process of starting up the medical device. During the booting, the medical device typically is powered on, and a medical application software is loaded such that the medical application software can be executed for operating the medical device.

A medical device of this kind may for example be an infusion device, such as a volumetric (peristaltic) infusion pump or a syringe infusion pump. The medical application herein may serve to control an administration operation of the medical device, such as the operation of a pumping mechanism of the medical device. For example, WO01/52935 A1 discloses such an infusion pump.

During the process of booting, a bootloader software (also commonly denoted, in short, as bootloader) stored typically at a predefined address in a non-volatile memory , for example a Flash memory or a so-called XIP ("Execute in place") memory, is executed, the bootloader software allowing for example to access other types of memory. The bootloader serves to load other software applications, in particular an application serving to control operation of the medical device, denoted herein as medical application software. Once the medical application software is loaded into the medical device's main memory, it is executed and from there on (in the sense of an operating system) controls operation of the medical device.

A bootloader conventionally is for example stored at a predefined, fixed memory address in a non-volatile memory and is executed at the starting up of the medical device for loading the medical application software. Because the bootloader is executed from the non-volatile memory it is stored in, an updating of the bootloader is generally not easily possible, because the bootloader cannot update itself within the non-volatile memory from which it is executed.

To allow an updating of a bootloader, conventionally therefore a two-stage bootloading process is employed, a primary bootloader being initially executed to load a secondary bootloader, which then loads the medical application software. Within the process of loading the secondary bootloader the primary bootloader may update the secondary bootloader, and the secondary bootloader within the process of loading the medical application software may update the medical application software, such that the secondary bootloader and the medical application software are upgradable.

Such two-stage bootloading process however requires an increased storage space. A two-stage bootloading process in addition may increase the booting time and may cause a rather complex booting sequence. In addition, a two-stage booting process may require a complex software code, which may potentially be prone to errors.

There hence is a desire to provide a method for booting a medical device and a medical device which allow, in an easy way, an upgrading of a bootloader software while reducing storage requirements and allowing for a straightforward booting sequence.

This object is achieved by means of a method comprising the features of claim 1.

The first memory is a non-volatile memory which in particular allows a software execution, for example a Flash memory.

The second memory, in turn, is a volatile memory, such as a random- access memory (in short: RAM). Alternatively, the second memory may be a so-called XIP memory, XIP being an abbreviation for "execute in place", such XIP memory being a non-volatile memory allowing for a direct execution of software that is installed in the XIP memory, in the instant case the bootloader.

A bootloader which is executed at the start-up of a medical device is stored, typically, at a predefined address in a first memory, which is a non-volatile memory. Because the bootloader cannot update itself immediately within the first memory, during execution of the bootloader software an instance of the bootloader software or a portion of the bootloader software is loaded into a second memory different from the first memory, such that an instance of the bootloader software or said portion of the bootloader software is transferred to the second memory different from the first memory. Hence, multiple instances of the bootloader software or said portion of the bootloader software exist, which makes it possible to alter the bootloader software or said portion of the bootloader software within the first memory and hence to update the bootloader software within the first memory.

The loading of the bootloader software or the portion of the bootloader software into the second memory herein takes place at an initial step of the booting sequence carried out by the bootloader. Hence, prior to executing steps relating to the actual loading of a medical application software for operating the medical device an updating of the bootloader software may be carried out, such that the actual loading of the medical application software then, after completion of the updating, may be carried out according to the updated bootloader software.

For the updating, the bootloader software or said portion of the bootloader software is executed from the second memory. Once the instance of the bootloader software or the portion of the bootloader software is loaded to the second memory, the execution jumps to the second memory and continues execution based on the instance of the bootloader software or the portion of the bootloader software loaded to the second memory.

Within the further processing, then, a software update of the bootloader software or said portion of the bootloader software is received and stored in the first memory, such that the bootloader software in the first memory is upgraded according to the available software update.

During the booting sequence, the bootloader software as a whole may be loaded to the second memory, for example a volatile random-access memory or a non-volatile XIP memory. If only a portion of the bootloader software shall be updated, only such portion of the bootloader software may be loaded into the second memory, hence reducing a required memory space in the second memory.

Generally, it is defined (permanently) by configuration at implementation whether to load the whole bootloader software into the second memory in order to allow updating of the entire bootloader software, or to load only a (defined) portion of the bootloader software into the second memory in order to allow updating of only the portion of the bootloader software.

By loading an instance of the bootloader software or the portion of the bootloader software into a second memory, an upgrading of the bootloader software in the first, non-volatile memory becomes possible. Once the bootloader software has been updated, the further execution of the bootloader software (potentially after a reset, i.e., a reboot of the system) may take place according to the updated bootloader software, and the loading of the actual medical application software may take place according to the updated bootloader software.

Hence, an updating of the bootloader software within a single-stage bootloading sequence is possible, reducing the overall complexity of the bootloading sequence. Also, because the bootloader software is designed to cause a booting of the medical device in a single stage, code complexity can be reduced, thus reducing the memory requirements and also a risk for errors in the code.

Because the bootloader uses less memory, a microcontroller on for example a processing board configured to execute the bootloader software may have reduced memory requirements, such that, e.g., a microcontroller having less internal memory may be employed.

The step of storing the software update of the bootloader software or said portion of the bootloader software in the first memory includes the erasing of a prior version of the bootloader software or said portion of the bootloader software in the first memory. Hence, the earlier version of the bootloader software or the portion of the bootloader software is deleted and replaced by the updated version of the bootloader software or the portion of the bootloader software.

In one embodiment, the step of storing the software update of the bootloader software or the portion of the bootloader software in the first memory furthermore includes the storing of the software update of the bootloader software or the portion of the bootloader software in a bootloader storage area of the first memory. From the bootloader storage area the software update of the bootloader software or the portion of the bootloader software is copied into a bootloader running area of the first memory, such that subsequently the bootloader software may be executed in the bootloader running area making use of its updated version. To provide a bootloader storage area may have the advantage that upgrading issues are avoided, especially in case a supply power is lost while upgrading.

Both the bootloader storage area and the bootloader running area herein may be part of a non-volatile memory, such that after a rebooting of the system the bootloader software is available for execution in the bootloader running area for starting up the medical device.

In one embodiment, after storing the software update of the bootloader software or the portion of the bootloader software in the first memory, the medical device is automatically rebooted. Hence, after updating the bootloader software, the booting process is started anew, such that the actual booting and in particular the loading of the medical application software takes place according to the updated bootloader software after rebooting the medical device.

In one embodiment, during execution of the bootloader software it is checked whether a software update of a medical application is available. If this is the case, the software update of the medical application software is received and stored in the first memory, in particular a non-volatile memory. Hence, by executing the bootloader software, not only the medical application software is loaded for execution, but also an updating of the medical application software may be carried out. For this, it is checked whether an update is available and, if yes, the update is carried out by storing the software update in the first memory and hence overwriting the prior medical application software.

The updating of the medical application software may take place sequentially (or possibly in parallel) to the updating of the bootloader software. In case of a sequential update, the bootloader software is updated first, and an updating of the medical application software then takes place only, e.g., after rebooting the system upon completion of the updating of the bootloader software.

The software update herein may relate to the entire medical application software or to a portion of the medical application software. Hence, a software update may replace the full medical application software stored previously or a portion of the medical application software.

The prior medical application software stored in the first memory hence is erased and replaced by the software update, such that on further execution of the bootloader the updated version of the medication application software is loaded and executed by the medical device.

In one embodiment, the storing of the software update of the medical application software includes the storing of the software update of the medical application software in an application storage area of the first memory and a subsequent copying of the software update of the medical application software into an application running area of the first memory for a subsequent execution. Hence, the software update of the medical application software is received and stored in the storage area reserved for the application. For execution, then, an instance of the medical application software is copied into the running area, from which it may then be executed.

Both the application storage area and the application running area herein may be part of a non-volatile memory.

Within the execution of the bootloader software, in addition, the integrity of the medical application software after updating may be checked. Hence, within the course of loading the medical application software it is checked whether the medical application software is ready to be executed or whether the medical application software is in any way corrupted, for example by a newly received software update, such that execution should be prohibited.

In one embodiment, the medical application software is executed following the execution of the bootloader software. The medical application software, after it is loaded by the bootloader software, herein is executed in its updated version, such that by executing the bootloader software it is ensured that the newest available version of the medical application software is loaded and executed during operation of the medical device.

For initiating the execution, the bootloader software may for example cause an execution of the medical application software within the non-volatile memory in which the medical application software is stored, and the medical application software is hence executed from the non-volatile memory for controlling operation of the medical device, for example for controlling an administration operation for administering a medical fluid, such as a medication or the like, to a patient, in particular in case the medical device is formed by an infusion pump such as a volumetric (peristaltic) infusion pump or a syringe infusion pump.

In one embodiment, the medical device comprises a first board and a second board, each processing board having at least one processor for carrying out computing operations.

The first board may for example be a so-called delivery board in case the medical device is an infusion device, such delivery board being configured to control operation of the medical device, for example operation of a pumping mechanism of an infusion pump. The bootloader software herein is executed on the first board for loading a medical application software functioning as an operating system for the first, delivery board.

The second board in turn may be for example a so-called HMI board (HMI for human machine interface), the second board for example running a conventional operating system, such as an embedded operation system, e.g. a Linux operating system, and being configured to provide HMI functions, such as a control of a display device and an input device of the medical device. Updates of the bootloader software and/or the medical application software may herein be provided by the second board to the first board, such that during booting of the system software updates of the bootloader software and/or the medical application software are received by the first board from the second board, for example by a communication between the first port and the second port employing for example standard communication protocols, such as an SPI or UART protocol.

In an alternative embodiment, the medical device may have a (single) mainboard and may receive updates of the bootloader software and/or the medical application software from an external tool, e.g. a software tool installed on a computing device, or external device such as an external server.

The object is also achieved by means of a medical device comprising the features of claim 11.

The advantages and advantageous embodiments described above for the method equally apply also to the medical device, such that it shall be referred to the above in this respect.

The medical device may in particular be an infusion device, such as a volumetric (peristaltic) infusion pump or a syringe infusion pump serving to administer a medical fluid, such as a medication or a nutritional feeding solution to a patient.

The invention shall subsequently be described in more detail according to the embodiments shown in the figures. Herein:
- Fig. 1: shows a schematic drawing of a medical device in the shape of an infusion device;
- Fig. 2: shows a schematic drawing of a processing assembly and a memory device of a medical device;
- Fig. 3: shows a schematic drawing of a memory architecture of a non-volatile memory of the memory device, according to a conventional design;
- Fig. 4: shows a schematic flow diagram of a conventional two-stage booting sequence employing a primary bootloader and a secondary bootloader;
- Fig. 5: shows a schematic drawing of an embodiment of a modified memory architecture; and
- Fig. 6: shows a schematic flow diagram of a booting sequence employing only a single bootloader.

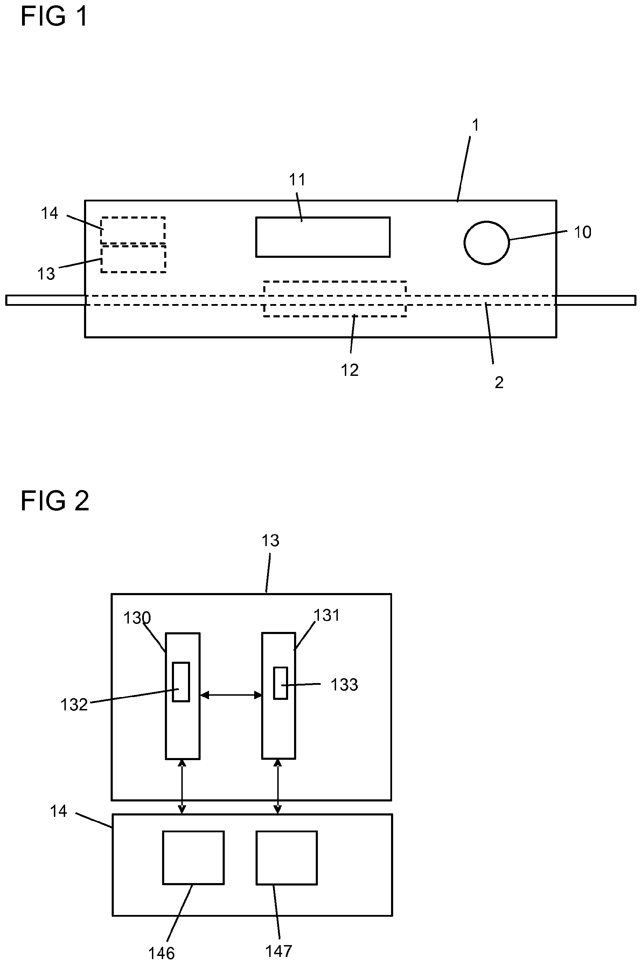

Fig. 1 shows a schematic drawing of a medical device 1 in the shape of an infusion device which serves to administer a medical fluid to a patient.

The medical device 1 in the shape of the infusion device may, in an exemplary embodiment, comprise a receptacle on which an infusion set 2 may be received, the infusion set 2 comprising an infusion line through which a medical fluid may be delivered towards the patient. When received on the medical device 1, the infusion set 2 is functionally connected to a pumping mechanism 12 of the medical device 1, the pumping mechanism 12 causing a delivery of a medical fluid through the infusion set 2 when the pumping mechanism 12 is operated.

The medical device 1 comprises a processing assembly 13, e.g. a microcontroller board, and a memory device 14, which together are configured to control operation of the medical device 1, in particular for performing an infusion operation for administering a medical fluid towards a patient.

The medical device 1 comprises an input device 10 in the shape of, e.g., a rotary knob which can be activated for example for programming an operation of the medical device 1, in particular for programming and setting up in infusion operation.

The medical device 1 furthermore comprises a display device 11 for displaying information, for example for displaying actual values of operational parameters such as the flow rate, a dosage or the dose rate. The display device 11 may output information about an ongoing operation of the medical device 1, for example in the context of an infusion operation, such as information relating to a progress of an infusion operation.

Referring now to Fig. 2, a processing assembly 13 in one embodiment comprises different processing boards 130, 131, the processing boards 130, 131 serving different functions within the medical device 1.

For example, a first board 130 may be denoted as delivery board and may serve to control operation of the medical device 1 relating to a pumping operation of the medical device 1. For example, the first board 130 may control a pumping mechanism 12 as illustrated in Fig. 1 and other control functions in relation to a pumping operation.

A second board 131 in turn may be denoted as HMI board and may serve to process an input and output of information. The second board 131 generally may control operation of for example the input device 10 and the display device 11 as illustrated in Fig. 1.

Each processing board 130, 131 comprises electrical and electronic components, in particular one or multiple processors 132, 133 in the shape of microcontrollers or the like.

A memory device 14 comprises different kinds of memory. In particular, a first memory 146 is a non-volatile memory. A second memory 147 in contrast is a volatile memory. A non-volatile memory may be external to the processing assembly 14, or may be an embedded memory within the processing assembly 13. A volatile memory is generally an internal memory embedded memory within the processing assembly 13, however may alternatively be an external memory.

During start-up of the medical device 1, both processing boards 130, 131 need to be booted. Herein, the first board 130 may for example run a dedicated operating system proprietary to a device manufacturer, whereas the second board 131 may run a standard, commercially available operating system such as for example a Linux operating system.

During the startup of the medical device 1, both processing boards 130, 131 are booted. Whereas the second board 131 may employ a standard booting sequence adapted to the operating system running on the second board 131, the first board 130 may employ a bootloader software which is dedicated to the operating system to be executed on the first board 130, subsequently denoted as medical application software.

For booting the first board 130, generally a bootloader software is employed which performs a booting sequence during which the medical application software functioning as operating system for the first board 130 is loaded and hence initiated for execution. As a bootloader may have bugs which potentially need to be fixed and as there may be the need to add new features to a bootloader software, there is a general desire to be able to update the bootloader software. As the bootloader software typically is stored at a predefined memory address in a non-volatile memory from which it directly is executed at the startup of the medical device 1, an updating however is not easily possible, because the bootloader software cannot erase itself in order to store and replace update functions for upgrading the bootloader software.

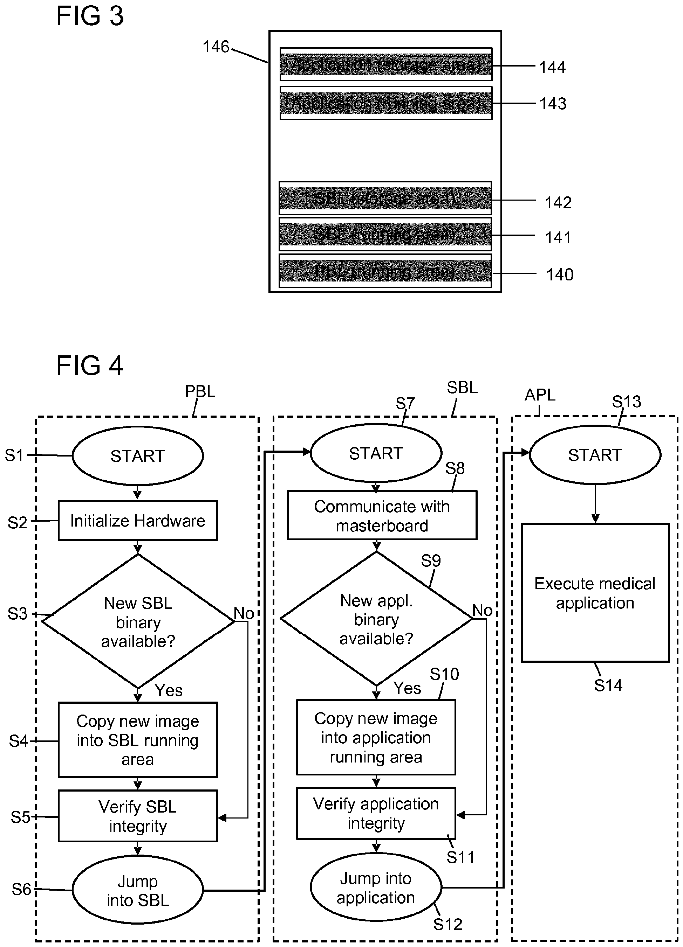

For this reason, within a conventional booting sequence a two-stage boot loading process may be employed, requiring a primary bootloader and a secondary bootloader, as this is schematically illustrated in Figs. 3 and 4.

Fig. 3 herein shows a possible memory architecture for such two-stage boot loading sequence, the memory architecture residing in a non-volatile memory 146 (internal or external to the processing assembly 13) and comprising a primary bootloader running area 140, a secondary bootloader running area 141, a secondary bootloader storage area 142, an application running area 143 and an application storage area 144. The primary bootloader herein is fixedly stored in the running area 140 and is executed from the running area 140. The primary bootloader, as shall subsequently be explained with reference to Fig. 4, serves to load the secondary bootloader, which then is executed to load the medical application software.

Referring now to Fig. 4, upon the startup of the medical device 1, in particular when powering the medical device 1 on, the primary bootloader (PBL) is executed (step S1) by carrying out the steps defined in the software of the primary bootloader in a sequential order. First, herein, the hardware of the medical device 1 is initialized (step S2), upon which a communication with the second board 131 (see Fig. 2) is carried out in order to receive a potentially new version of the secondary bootloader, which is stored (if available) in the secondary bootloader storage area 142, as illustrated in Fig. 3. If a new version of the secondary bootloader is available (step S3), the received new version of the secondary bootloader is copied into the secondary bootloader running area 141 (step S4), upon which the software integrity of the secondary bootloader is verified in step S5 and it is jumped, in case of a positive result in the verification step S5, into the secondary bootloader (step S6) for executing the secondary bootloader.

If in step S3 it is found that as a result of the communication with the second board 131 no new version of the secondary bootloader is available, it is directly stepped to step S5, hence verifying the software integrity of the already existing secondary bootloader, upon which it is jumped into the secondary bootloader (step S6).

Hence, execution of the secondary bootloader (SBL) starts (step S7), wherein, if a new version of the secondary bootloader has been received and loaded during execution of the primary bootloader (PBL), the execution of the secondary bootloader takes place according to the new, updated version of the secondary bootloader.

During execution of the secondary bootloader, the secondary bootloader communicates with the second board 131 (step S8) in order to receive, if available, a software update for the medical application software to be executed for operating the medical device 1. The software update for the medical application software herein is stored in the application storage area 144 (see Fig. 3).

If a software update for the medical application software has been received and hence is available (step S9), an instance of the medical application software is copied from the application storage area 144 to the application running area 143 (step S10), upon which the medical application software is checked for integrity (step S11). Following a positive result for the verification, it then is jumped into the medical application software (step S12), and the medical application software (APL) is executed (steps S13, S14).

With such two-stage booting sequence an updating of the secondary bootloader and the medical application software, which serves as an operating system for the first board 130 of the processing assembly 13 of the medical device 1, can be updated. As two booting stages are employed, the software code for implementing the booting sequence may be complex and hence prone to errors, and in addition significant memory space is required for storing and executing the booting sequence.

In order to reduce complexity of the booting sequence and to additionally reduce a memory requirement, it herein is proposed to employ a one-stage booting sequence using a single bootloader software, which serves to load the medical application software and in addition has a functionality to update itself.

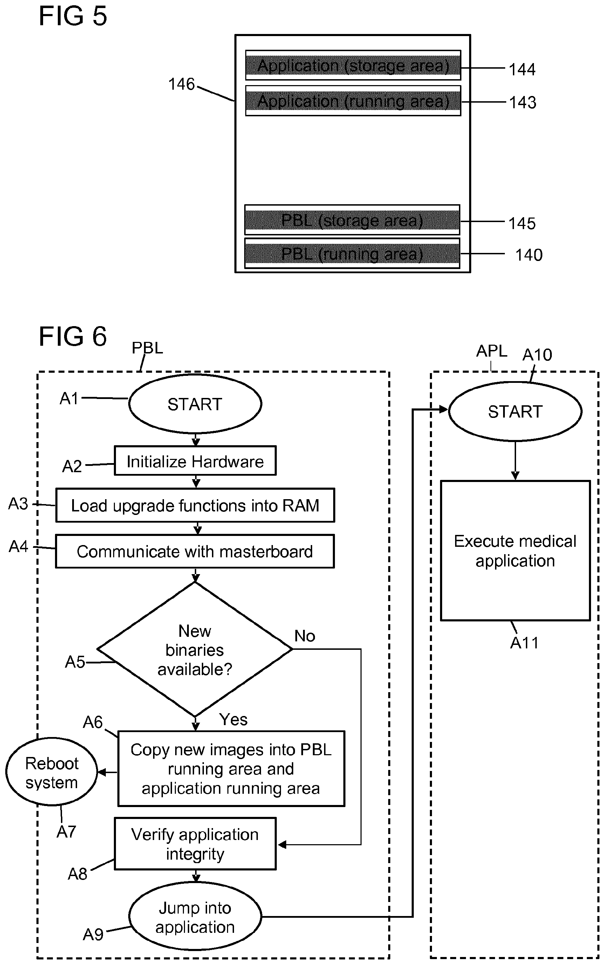

Referring now to Fig. 5, within a modified memory architecture of a non-volatile memory 146 a running area 140 and a storage area 145 for the bootloader software (PBL) are defined. In addition, a running area 143 and a storage area 144 for the medical application software exists.

Referring now to Fig. 6, the booting of the medical device 1 employing a bootloader software defining a single booting stage starts at step A1 for example when starting up the medical device 1, for example when switching the medical device 1 on. Initially, the hardware of the medical device 1 is initialized (step A2), upon which the bootloader software as a whole or such functions of the bootloader software which potentially are subject to an update are loaded into a volatile memory (RAM) or alternatively into an XIP memory. Hence, the bootloader software stored in the bootloader storage area 145 or a portion of the bootloader software which may be subject to an update is loaded into another memory, which is separate from the bootloader storage area 145 and allows for an execution of the bootloader software.

Following the loading of the bootloader software as a whole or a portion of the bootloader software into the other memory in step A3, it is jumped to the memory into which the bootloader software or the portion of the bootloader software has been loaded for executing the bootloader software or the portion of the bootloader software in such other memory location. The bootloader software now communicates with the second board 131 (see Fig. 2) in order to receive a software update for the bootloader software or at least particular update functions of the bootloader software (step A4). The software update for the bootloader software is loaded into the bootloader storage area 145, overwriting the code of the bootloader which previously has been stored in the bootloader storage area 145 and hence erasing a prior version of the bootloader software or at least a portion of the bootloader software which is subject to updating.

Alternatively, it is possible to copy the received software update directly into the bootloader running area 140 (hence eliminating the step of storing the software update in a bootloader storage area 145 first), in particular if it is ensured that a safe hardware and a safe power supply is available.

Also in step A4, if available a software update for the medical application software is received from the second board 131 (or from an external tool using a similar or the same communication protocol) and is stored in the application storage area 144, overwriting a previous version of the medical application software and hence updating the medical application software in the application storage area 144.

In step A5 it is checked whether software updates for the bootloader software and the medical application software have been received and hence are available. If this is the case, instances of the bootloader software and the medical application software are copied into the bootloader running area 140 respectively the application running area 143 (step A6), upon which a reboot of the system is initiated, hence starting the booting process anew (step A7).

If a reboot of the system is carried out, the same steps as in the first run through the booting sequence are carried out (steps A1 to A4), wherein now typically no software updates are received, such that in step A5 it is found that no software updates are available. It hence is jumped to step A8, in which the integrity of the medical application software is verified.

If the integrity check in step A8 is successful, in step A9 it is jumped into the medical application software, and an execution of the medical application software is carried out (steps A10, A11).

By loading the bootloader software as a whole or a portion of the bootloader software which potentially is subject to an update into another, volatile memory (RAM) or an XIP memory in step A3, and by subsequently executing the bootloader from the other memory location, it becomes possible to erase the bootloader software as a whole or portions of the bootloader software in the bootloader running area 140, such that an updating of the bootloader software is possible within the one-stage booting process. Hence, the loading of the bootloader software or the portion of the bootloader software to another memory allows reducing complexity of the booting sequence in that a secondary bootloader can be avoided while still allowing for an updating of the bootloader software.

Hence, memory requirements may be reduced, and due to a reduced complexity of the booting sequence a risk for software bugs may be reduced, as the software code for the bootloader software may overall be simpler.

The first board 130 receives software updates for the bootloader software and/or the medical application software from the second board 131. The processing boards 130, 131 are in communication connection with each other, the second board 131 for example employing a standard communication protocol for transferring software updates towards the first board 130, such as an UART or an SPI protocol.

The idea of the invention is not limited to the embodiments described above, but may be implemented in a different fashion.

A medical device may be an infusion device such as a volumetric (peristaltic) infusion pump or a syringe infusion pump. The medical device however may be another device to be operated in a healthcare environment such as a hospital, for example any device which serves a function for example for administering medication or any other medical solution to a patient.

### List of Reference Numerals

- 1: Medical device
- 10: Input device
- 11: Display device
- 12: Pumping mechanism
- 13: Processing assembly
- 130, 131: Processing board
- 132, 133: Processor
- 14: Memory device
- 140: PBL (running area)
- 141: SBL (running area)
- 142: SBL (storage area)
- 143: Application (running area)
- 144: Application (storage area)
- 145: PBL (storage area)
- 146: Non-volatile memory
- 147: Volatile memory
- 2: Infusion set
- A1-A11: Steps
- APL: Medical application software
- PBL: Bootloader software program
- S1-S14: Steps
- SBL: Secondary bootloader software

## Claims

1. Method for booting a medical device (1), comprising:
executing a bootloader software, PBL, stored in a first memory (146) of a memory device (14) of the medical device (1) for booting the medical device (1), wherein the bootloader software, PBL, is directly executed at the startup of the medical device (1) to initialize the hardware of the medical device (1) and to start execution of a medical application software, APL;
loading, during execution of the bootloader software, PBL, in the first memory (146), the bootloader software, PBL, or a portion of the bootloader software, PBL, into a second memory (147) of the memory device (14) of the medical device (1) which is different from the first memory (146);
executing the bootloader software, PBL, or said portion of the bootloader software, PBL, from the second memory (147);
receiving a software update of the bootloader software, PBL, or said portion of the bootloader software, PBL; and
storing the software update of the bootloader software, PBL, or said portion of the bootloader software, PBL, in the first memory (146),
wherein storing of the software update of the bootloader software, PBL, or said portion of the bootloader software, PBL, in the first memory (146) includes erasing a prior version of the bootloader software, PBL, or said portion of the bootloader software, PBL,
and wherein the first memory (146) is a non-volatile memory and the second memory (147) is a volatile memory.

2. Method according to claim 1, **characterized in that** the second memory (147) is a RAM memory or an XIP memory.

3. Method according to claim 1 or 2, **characterized in that** said storing of the software update of the bootloader software, PBL, or said portion of the bootloader software, PBL, in the first memory (146) includes: storing the software update of the bootloader software, PBL, or said portion of the bootloader software, PBL, in a bootloader storage area (145) of the first memory (146) and copying the software update of the bootloader software, PBL, or said portion of the bootloader software, PBL, into a bootloader running area (140) of the first memory (146) for a subsequent execution.

4. Method according to one of the preceding claims, **characterized in that**, following the storing of the software update of the bootloader software, PBL, or said portion of the bootloader software, PBL, in the first memory (146), the medical device (1) is rebooted.

5. Method according to one of the preceding claims, **characterized by,** during execution of the bootloader software, PBL, checking if a software update of the medical application software, APL, is available; and, if a software update of the medical application software, APL, is available, receiving said software update of the medical application software, APL, and storing the software update of the medical application software, APL, in the first memory (146).

6. Method according to claim 5, **characterized in that** said storing of the software update of the medical application software, APL, in the first memory (146) includes: erasing a prior version of the medical application software, APL.

7. Method according to claim 5 or 6, **characterized in that** said storing of the software update of the medical application software, APL, includes: storing the software update of the medical application software, APL, in an application storage area (144) of the first memory (146) and copying the software update of the medical application software, APL, into an application running area (143) of the first memory (146) for a subsequent execution.

8. Method according to one of claims 5 to 7, **characterized by,** during execution of the bootloader software, PBL, verifying the integrity of the medical application software, APL.

9. Method according to one of claims 5 to 8, **characterized by** executing, after competition of execution of the bootloader software, PBL, the medical application software, APL, using the software update of the medical application software, APL.

10. Method according to one of the preceding claims, **characterized in that** the medical device (1) comprises a first board (130) and a second board (131), each board having at least one processor (132, 133), wherein the bootloader software, PBL is executed on the first board (130) and the software update of the bootloader software, PBL is received from the second board (131).

11. Medical device (1), comprising:
a memory device (14) having a first memory (146) and a second memory (147); and
a processing assembly (13) configured to execute a bootloader software, PBL stored in the first memory (146) of the memory device (14) of the medical device (1) for booting the medical device (1), wherein the processing assembly (13) is configured to execute the bootloader software, PBL, directly at the startup of the medical device (1) to initialize the hardware of the medical device (1) and to start execution of a medical application software, APL;
wherein the processing assembly (1) is configured to load, during execution of the bootloader software, PBL, in the first memory (146), the bootloader software, PBL, or a portion of the bootloader software, PBL, into the second memory (147) of the memory device (14) of the medical device (1) which is different from the first memory (146),
execute the bootloader software, PBL, or said portion of the bootloader software, PBL, from the second memory (147),
receive a software update of the bootloader software, PBL, or said portion of the bootloader software, PBL, and
store the software update of the bootloader software, PBL, or said portion of the bootloader software, PBL, in the first memory (146),
wherein storing of the software update of the bootloader software, PBL, or said portion of the bootloader software, PBL, in the first memory (146) includes erasing a prior version of the bootloader software, PBL, or said portion of the bootloader software, PBL,
wherein the first memory (146) is a non-volatile memory and the second memory (147) is a volatile memory.

## Patentansprüche

1. Verfahren zum Booten einer medizinischen Vorrichtung (1), umfassend:
Ausführen einer Bootloader-Software, PBL, die in einem ersten Speicher (146) einer Speichervorrichtung (14) der medizinischen Vorrichtung (1) zum Booten der medizinischen Vorrichtung (1) gespeichert ist, wobei die Bootloader-Software, PBL, unmittelbar beim Hochfahren der medizinischen Vorrichtung (1) ausgeführt wird, um die Hardware der medizinischen Vorrichtung (1) zu initialisieren und die Ausführung einer medizinischen Anwendungssoftware, APL, zu starten;
Laden, während der Ausführung der Bootloader-Software, PBL, in dem ersten Speicher (146), der Bootloader-Software, PBL, oder eines Teils der Bootloader-Software, PBL, in einen zweiten Speicher (147) der Speichervorrichtung (14) der medizinischen Vorrichtung (1), der sich von dem ersten Speicher (146) unterscheidet;
Ausführen der Bootloader-Software, PBL, oder des Teils der Bootloader-Software, PBL, aus dem zweiten Speicher (147);
Empfangen eines Software-Updates der Bootloader-Software, PBL, oder des Teils der Bootloader-Software, PBL; und
Speichern des Software-Updates der Bootloader-Software, PBL, oder des Teils der Bootloader-Software, PBL, in dem ersten Speicher (146),
wobei das Speichern des Software-Updates der Bootloader-Software, PBL, oder des Teils der Bootloader-Software, PBL, in dem ersten Speicher (146) das Löschen einer früheren Version der Bootloader-Software, PBL, oder des Teils der Bootloader-Software, PBL, beinhaltet,
und wobei der erste Speicher (146) ein nichtflüchtiger Speicher ist und der zweite Speicher (147) ein flüchtiger Speicher ist.

2. Verfahren nach Anspruch 1, **dadurch gekennzeichnet, dass** der zweite Speicher (147) ein RAM-Speicher oder ein XIP-Speicher ist.

3. Verfahren nach Anspruch 1 oder 2, **dadurch gekennzeichnet, dass** Speichern des Software-Updates der Bootloader-Software, PBL, oder des Teils der Bootloader-Software, PBL, in dem ersten Speicher (146) umfasst:
Speichern des Software-Updates der Bootloader-Software, PBL, oder des Teils der Bootloader-Software, PBL, in einem Bootloader-Speicherbereich (145) des ersten Speichers (146) und Kopieren des Software-Updates der Bootloader-Software, PBL, oder des Teils der Bootloader-Software, PBL, in einen Bootloader-Laufbereich (140) des ersten Speichers (146) für eine nachfolgende Ausführung.

4. Verfahren nach einem der vorhergehenden Ansprüche, **dadurch gekennzeichnet, dass** nach dem Speichern des Software-Updates der Bootloader-Software, PBL, oder des Teils der Bootloader-Software, PBL, in dem ersten Speicher (146), die medizinische Vorrichtung (1) neu gebootet wird.

5. Verfahren nach einem der vorhergehenden Ansprüche, **gekennzeichnet durch,** während der Ausführung der Bootloader-Software, PBL, Prüfen, ob ein Software-Update der medizinischen Anwendungssoftware, APL, verfügbar ist; und, falls ein Software-Update der medizinischen Anwendungssoftware, APL, verfügbar ist, Empfangen des Software-Updates der medizinischen Anwendungssoftware, APL, und Speichern des Software-Updates der medizinischen Anwendungssoftware, APL, in dem ersten Speicher (146).

6. Verfahren nach Anspruch 5, **dadurch gekennzeichnet, dass** das Speichern des Software-Updates der medizinischen Anwendungssoftware, APL, in dem ersten Speicher (146) beinhaltet: Löschen einer früheren Version der medizinischen Anwendungssoftware, APL.

7. Verfahren nach Anspruch 5 oder 6, **dadurch gekennzeichnet, dass** das Speichern des Software-Updates der medizinischen Anwendungssoftware, APL, beinhaltet:
Speichern dew Software-Updates der medizinischen Anwendungssoftware, APL, in einem Anwendungsspeicherbereich (144) des ersten Speichers (146) und Kopieren des Software-Updates der medizinischen Anwendungssoftware, APL, in einen Anwendungslaufbereich (143) des ersten Speichers (146) für eine nachfolgende Ausführung.

8. Verfahren nach einem der Ansprüche 5 bis 7, **gekennzeichnet durch,** während der Ausführung der Bootloader-Software, PBL, Verifizieren der Integrität der medizinischen Anwendungssoftware, APL.

9. Verfahren nach einem der Ansprüche 5 bis 8, **gekennzeichnet durch** Ausführen, nach dem Ausführungswettbewerb der Bootloader-Software, PBL, der medizinischen Anwendungssoftware, APL, unter Verwendung des Software-Updates der medizinischen Anwendungssoftware, APL.

10. Verfahren nach einem der vorhergehenden Ansprüche, **dadurch gekennzeichnet, dass** die medizintechnische Vorrichtung (1) eine erste Platine (130) und eine zweite Platine (131) umfasst, wobei jede Platine mindestens einen Prozessor (132, 133) aufweist, wobei die Bootloader-Software, PBL, auf der ersten Platine (130) ausgeführt wird und das Software-Update der Bootloader-Software, PBL, von der zweiten Platine (131) empfangen wird.

11. Medizinische Vorrichtung (1), umfassend:
eine Speichervorrichtung (14)mit einen ersten Speicher (146) und einen zweiten Speicher (147); und
eine Verarbeitungsbaugruppe (13), ausgelegt zum Ausführen einer Bootloader-Software, PBL, die in dem ersten Speicher (146) der Speichervorrichtung (14) der medizinischen Vorrichtung (1) gespeichert ist, zum Booten der medizinischen Vorrichtung (1), wobei die Verarbeitungsbaugruppe (13) ausgelegt ist zum Ausführen der Bootloader-Software, PBL, direkt beim Hochfahren der medizinischen Vorrichtung (1), um die Hardware der medizinischen Vorrichtung (1) zu initialisieren und die Ausführung einer medizinischen Anwendungssoftware, APL, zu starten;
wobei die Verarbeitungsbaugruppe (1) ausgelegt ist zum Laden, während der Ausführung der Bootloader-Software, PBL, in dem ersten Speicher (146), der Bootloader-Software, PBL, oder eines Teils der Bootloader-Software, PBL, in den zweiten Speicher (147) der Speichervorrichtung (14) der medizinischen Vorrichtung (1), der sich von dem ersten Speicher (146) unterscheidet,
Ausführen der Bootloader-Software, PBL, oder des Teils der Bootloader-Software, PBL, aus dem zweiten Speicher (147),
Empfangen eines Software-Updates der Bootloader-Software, PBL, oder des Teils der Bootloader-Software, PBL, und
Speichern des Software-Updates der Bootloader-Software, PBL, oder des Teils der Bootloader-Software, PBL, in dem ersten Speicher (146),
wobei das Speichern des Software-Updates der Bootloader-Software, PBL, oder des Teils der Bootloader-Software, PBL, in dem ersten Speicher (146) das Löschen einer früheren Version der Bootloader-Software, PBL, oder des Teils der Bootloader-Software, PBL, beinhaltet,
wobei der erste Speicher (146) ein nichtflüchtiger Speicher ist und der zweite Speicher (147) ein flüchtiger Speicher ist.

## Revendications

1. Procédé d'amorçage d'un dispositif médical (1), comprenant :
l'exécution d'un logiciel d'amorçage, PBL, stocké dans une première mémoire (146) d'un dispositif de mémoire (14) du dispositif médical (1) pour amorcer le dispositif médical (1), dans lequel le logiciel d'amorçage, PBL, est exécuté directement au démarrage du dispositif médical (1) pour initialiser le matériel du dispositif médical (1) et pour démarrer l'exécution d'un logiciel d'application médicale, APL ;
le chargement, durant l'exécution du logiciel d'amorçage, PBL, dans la première mémoire (146), du logiciel d'amorçage, PBL, ou d'une partie du logiciel d'amorçage, PBL, dans une seconde mémoire (147) du dispositif de mémoire (14) du dispositif médical (1) qui est différente de la première mémoire (146) ;
l'exécution du logiciel d'amorçage, PBL, ou de ladite partie du logiciel d'amorçage, PBL, à partir de la seconde mémoire (147) ;
la réception d'une mise à jour logicielle du logiciel d'amorçage, PBL, ou de ladite partie du logiciel d'amorçage, PBL ; et
le stockage de la mise à jour logicielle du logiciel d'amorçage, PBL, ou de ladite partie du logiciel d'amorçage, PBL, dans la première mémoire (146),
dans lequel le stockage de la mise à jour logicielle du logiciel d'amorçage, PBL, ou de ladite partie du logiciel d'amorçage, PBL, dans la première mémoire (146) comprend l'effacement d'une version antérieure du logiciel d'amorçage, PBL, ou de ladite partie du logiciel d'amorçage, PBL,
et dans lequel la première mémoire (146) est une mémoire non volatile et la seconde mémoire (147) est une mémoire volatile.

2. Procédé selon la revendication 1, **caractérisé en ce que** la seconde mémoire (147) est une mémoire RAM ou une mémoire XIP.

3. Procédé selon la revendication 1 ou 2, **caractérisé en ce que** ledit stockage de la mise à jour logicielle du logiciel d'amorçage, PBL, ou de ladite partie du logiciel d'amorçage, PBL, dans la première mémoire (146) comprend : le stockage de la mise à jour logicielle du logiciel d'amorçage, PBL, ou de ladite partie du logiciel d'amorçage, PBL, dans une zone de stockage de logiciel d'amorçage (145) de la première mémoire (146) et la copie de la mise à jour logicielle du logiciel d'amorçage, PBL, ou de ladite partie du logiciel d'amorçage, PBL, dans une zone d'exécution de logiciel d'amorçage (140) de la première mémoire (146) en vue d'une exécution ultérieure.

4. Procédé selon l'une des revendications précédentes, **caractérisé en ce que,** suite au stockage de la mise à jour logicielle du logiciel d'amorçage, PBL, ou de ladite partie du logiciel d'amorçage, PBL, dans la première mémoire (146), le dispositif médical (1) est réamorcé.

5. Procédé selon l'une des revendications précédentes, **caractérisé par**, durant l'exécution du logiciel d'amorçage, PBL, la vérification si une mise à jour logicielle du logiciel d'application médicale, APL, est disponible; et, si une mise à jour logicielle du logiciel d'application médicale, APL, est disponible, la réception de ladite mise à jour logicielle du logiciel d'application médicale, APL, et le stockage de la mise à jour logicielle du logiciel d'application médicale, APL, dans la première mémoire (146).

6. Procédé selon la revendication 5, **caractérisé en ce que** ledit stockage de la mise à jour logicielle du logiciel d'application médicale, APL, dans la première mémoire (146) comprend : l'effacement d'une version antérieure du logiciel d'application médicale, APL.

7. Procédé selon la revendication 5 ou 6, **caractérisé en ce que** ledit stockage de la mise à jour logicielle du logiciel d'application médicale, APL, comprend : le stockage de la mise à jour logicielle du logiciel d'application médicale, APL, dans une zone de stockage d'application (144) de la première mémoire (146) et la copie de la mise à jour logicielle du logiciel d'application médicale, APL, dans une zone d'exécution d'application (143) de la première mémoire (146) en vue d'une exécution ultérieure.

8. Procédé selon l'une des revendications 5 à 7, **caractérisé par**, durant l'exécution du logiciel d'amorçage, PBL, la vérification de l'intégrité du logiciel d'application médicale, APL.

9. Procédé selon l'une des revendications 5 à 8, **caractérisé par** l'exécution, après concurrence de l'exécution du logiciel d'amorçage, PBL, du logiciel d'application médicale, APL, à l'aide de la mise à jour logicielle du logiciel d'application médicale, APL.

10. Procédé selon l'une des revendications précédentes, **caractérisé en ce que** le dispositif médical (1) comprend une première carte (130) et une seconde carte (131), chaque carte comportant au moins un processeur (132, 133), dans lequel le logiciel d'amorçage, PBL, est exécuté sur la première carte (130) et la mise à jour logicielle du logiciel d'amorçage, PBL, est reçue à partir de la seconde carte (131).

11. Dispositif médical (1), comprenant :
un dispositif de mémoire (14) comportant une première mémoire (146) et une seconde mémoire (147) ; et
un ensemble de traitement (13) configuré pour exécuter un logiciel d'amorçage, PBL, stocké dans la première mémoire (146) du dispositif de mémoire (14) du dispositif médical (1) pour amorcer le dispositif médical (1), dans lequel l'ensemble de traitement (13) est configuré pour exécuter le logiciel d'amorçage, PBL, directement au démarrage du dispositif médical (1) pour initialiser le matériel du dispositif médical (1) et pour démarrer l'exécution d'un logiciel d'application médicale, APL ;
dans lequel l'ensemble de traitement (1) est configuré pour charger, durant l'exécution du logiciel d'amorçage, PBL, dans la première mémoire (146), le logiciel d'amorçage, PBL, ou une partie du logiciel d'amorçage, PBL, dans la seconde mémoire (147) du dispositif de mémoire (14) du dispositif médical (1) qui est différente de la première mémoire (146),
exécuter le logiciel d'amorçage, PBL, ou ladite partie du logiciel d'amorçage, PBL, à partir de la seconde mémoire (147),
recevoir une mise à jour logicielle du logiciel d'amorçage, PBL, ou de ladite partie du logiciel d'amorçage, PBL, et
stocker la mise à jour logicielle du logiciel d'amorçage, PBL, ou de ladite partie du logiciel d'amorçage, PBL, dans la première mémoire (146),
dans lequel le stockage de la mise à jour logicielle du logiciel d'amorçage, PBL, ou de ladite partie du logiciel d'amorçage, PBL, dans la première mémoire (146) comprend l'effacement d'une version antérieure du logiciel d'amorçage, PBL, ou de ladite partie du logiciel d'amorçage, PBL,
dans lequel la première mémoire (146) est une mémoire non volatile et la seconde mémoire (147) est une mémoire volatile.
